# EUROPEAN PATENT SPECIFICATION

(11) **EP 0 453 008 B1**
(45) Date of publication and mention of the grant of the patent: **09.11.1994**
(21) Application number: 91200577.4
(22) Date of filing: 15.03.1991
(51) Int. Cl.: A61M 25/00

(54) **Angiographic catheter**
Angiographiekatheter
Cathéter d'angiographie

(30) Priority: 09.04.1990 NL 9000833
(43) Date of publication of application: 23.10.1991
(73) Proprietor: CORDIS EUROPA N.V., NL-9301 LJ Roden (NL)
(72) Inventor: Griep, Wilhelmus Antonius Maria, NL-9301 PK Roden (NL)
(74) Representative: 't Jong, Bastiaan Jacobus

(56) References cited:
- EP-A- 0 117 093
- EP-A- 0 273 618
- EP-A- 0 303 487
- US-A- 4 735 620

## Description

The invention relates to an angiographic catheter comprising a stiff hose-like basic body and a less stiff hose-like end part connected to one end of this basic body. A number of openings are arranged in the wall of the end part and the free end likewise comprises an outlet opening.

During angiographic examination such a catheter is inserted via the blood vessel system of a patient such that the end part enters the heart and/or the aorta. Via a central channel in the catheter a contrast fluid can be injected out of the openings, whereby the heart and/or the aorta thereof can be made visible in a radiological laboratory.

When not loaded the end part has a curved shape which is specific to the place where the end part of the catheter has to be positioned in the blood vessel system. As the catheter is inserted the end part is kept straightened using a guide wire.

The end part must be sufficiently stiff to preserve its curved shape during injection of the contrast fluid. This is of great clinical importance since the correct form prevents the catheter from vibrating during injection of the contrast fluid and moreover prevents the liquid flowing outward with force from the openings damaging the walls of the blood vessels.

However, a stiff end part again has the drawback that insertion and withdrawal of the catheter must take place with additionally great care in order to prevent damage to the blood vessels.

The invention has for its object to provide an angiographic catheter of the type described in the preamble of present Claim 1 and which is known from US-A-4 735 620, the end part of which preserves its shape well during use, but which can still be inserted into and removed from the patient safely.

This is achieved with an angiographic catheter according to the invention in that the end part is assembled from at least two hose portions of material with different stiffness mutually connecting in lengthwise direction. By thus constructing the end part from portions of different stiffness, it can be achieved in favorable manner that the portions of the end part which determine the form-retaining capacity during use have a relatively great stiffness, thus well ensuring this form-retaining capacity, while the portions which potentially cause the greatest risk of damage to the vessel walls are manufactured from soft, flexible material.

In particular, the catheter according to the invention is preferably embodied such that the stiffness of the material of the hose portions decreases in the direction of the basic body to the free end. The portion close to the free end, which forms the foremost part of the catheter during insertion of the catheter into the patient, thus has the smallest stiffness and so the smallest risk of damaging the vessel wall.

The invention is particularly suitable for use in a catheter of the so-called "pigtail" type. The curved shape of the end part here comprises a straight portion connecting onto the basic body and a circular portion curved substantially through more than 270° connecting to the straight portion.

A catheter of this type is embodied in a favorable manner such that a first relatively stiff hose portion of the end part extends from the basic body through substantially 180° of the circular curved part and a second markedly more flexible hose portion extends over the remaining portion of the curved part, that is, to the end.

A gradual transition in the stiffness is achieved through the material transitions from the basic body to the first hose portion of the end part and from the first to the second hose portion. This is favorable in order to be able to manipulate the catheter well without this quickly forming sharp ridges.

The invention is particularly important for thin catheters having a thin wall.

Further advantages and features of the invention will become apparent from the following description with reference to the annexed figures.

Fig. 1 shows a portion of a catheter according to the invention close to the end part thereof.

Fig. 2 shows a view corresponding with fig. 1 for elucidation of the method of manufacture thereof.

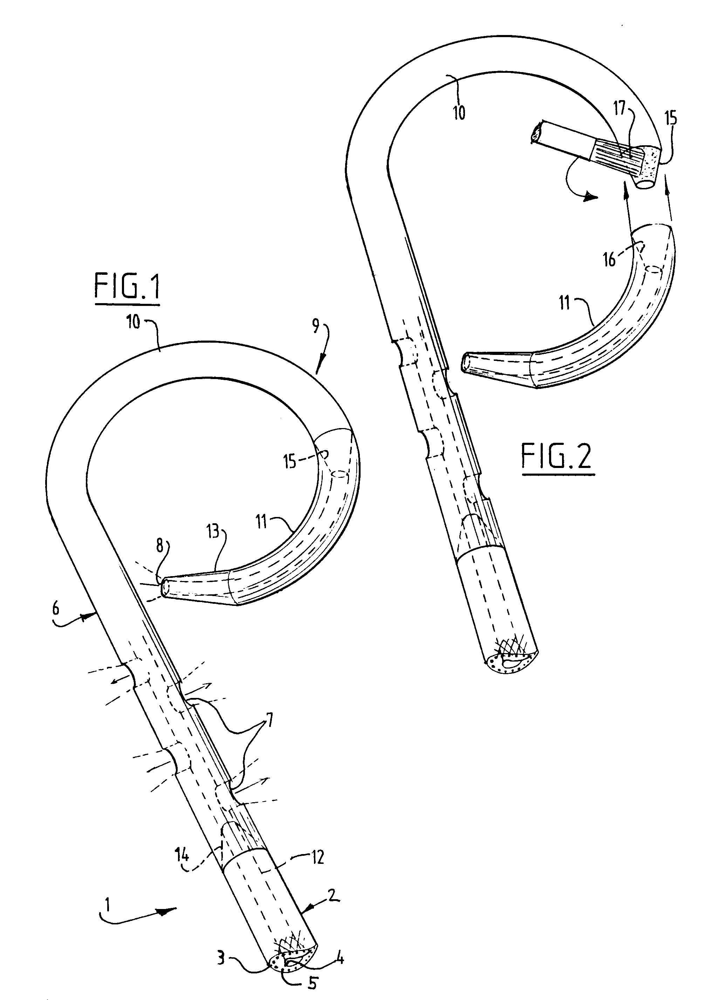

The catheter 1 shown in fig. 1 comprises a stiff hose-like basic body 2, only a small portion of which is shown. In the usual manner a coupling member is arranged on the end of the basic body 2 not shown for connection to a liquid injection device.

The basic body 2 of the catheter 1 consists of an outer layer 3 and an inner layer 4 of thermoplastic plastic. Embedded between these two layers 3, 4 is a reinforcing sheath 5 of braided wire. This basic body 2 is designed such that at great lengths of many tens of centimeters and with a very small diameter it has suitable properties, such as a sufficient torsion stiffness and pressure resistance in order respectively to be able to insert the catheter into a patient with good control and during use to press contrast fluid with considerable pressure into the end of the basic body provided with the coupling member.

The end of the catheter 1 for insertion into the patient comprises an end part 6 connected to the basic body 2 and having no braided sheath. Arranged in the wall of the end part 6 are a number of openings 7, while an end opening 8 is likewise present in the outermost end of the end part 6. These openings 7, 8 communicate with a central channel 12 in the catheter, through which the contrast fluid can be supplied.

The end part has a curved form 9 which depends on the desired positioning of the end part during examination of the patient. Shown in the figures is a catheter of the so-called "pigtail" type wherein the end part comprises a straight portion connecting to the basic body 2 and a circular portion curved substantially through more than 270° connecting to the straight portion.

As shown, the end part 6 is assembled from a first hose portion 10 and a second hose portion 11. The second hose portion 11 has a lesser stiffness than the first hose portion 10. In the preferred embodiment shown the first hose portion 10 extends from the basic body 2 through substantially 180° of the circular curved part 9. The second end portion 11 extends through the remaining portion of the curved part 9.

This embodiment of the end part 6 according to the invention achieves on the one hand that this end part retains its shape well so that the curved form 9 is preserved well during injection of contrast fluid. The thereby occurring reaction forces have the tendency to "bend out" the curved form 9. Because the first end portion 10 is manufactured from relatively stiff material, these reaction forces are taken up well.

The second hose portion 11 is of much more flexible material than the first hose portion 10. During insertion of the catheter this portion 11 forms the protruding front end of the catheter and because of its good flexibility the forces exerted on the walls of the blood vessels during insertion do not become so great that the blood vessel wall can suffer damage as a result. When this exerted force increases, the flexible hose portion 11 simply bends to the side.

The outermost end of the end part 6, close to the end opening 8, is given a point in the usual manner as indicated at 13. This also contributes to reducing the risk of damage.

As indicated by dashed lines at 14, the end part 6 is adhered fixedly to a bevelled end surface of the basic body 2. The two hose portions 10, 11 can be connected to each other in similar manner. As fig. 2 schematically indicates, the end 15 of the first hose portion 10 will be bevelled, whereafter adhesive is applied to the surface 15, as designated symbolically with the brush 17, and the end of the hose portion 11 is pushed over the bevelled surface 15 such that it is connected thereto.

It is noted that figure 2 serves only to illustrate the connection of the hose portions 10, 11. In reality the hose portions will of course already be connected to each other and the hose 10 will be connected to the basic body before the curved form 9 is arranged therein. A metal guide wire is inserted into the central channel 12 and the various hose portions are pushed over this wire up against each other and adhered fixedly to each other. Thereafter the outer surface of the end part is ground smooth, the curved form is arranged therein by plastic deformation and the openings are punched into the wall.

Instead of adhesive connections, other suitable forms of connection can of course be used, such as ultrasonic welding and the like.

The invention is not limited to a catheter of the "pigtail" type having an end part assembled from two hose portions. Catheters with differently formed end parts can also be embodied in a favorable manner according to the invention, wherein these end parts may also be assembled from more than two hose portions and the different stiffness of the hose portions chosen accordingly.

## Claims

1. Angiographic catheter comprising a stiff hose-like basic body (2) and a less stiff hose-like end part (6) connected to one end of this basic body, wherein this end part has a curved form, characterized in that said end part is assembled from at least two hose portions (10, 11) of material of different stiffness connected to one another in lengthwise direction.

2. Catheter as claimed in claim 1, wherein the stiffness of the material of the hose portions (10, 11) decreases in the direction of the basic body towards the free end.

3. Catheter as claimed in claim 2 of the "pigtail" type, wherein the curved form of the end part comprises a straight portion connecting to the basic body and a circular portion curved substantially through more than 270° connected to the straight portion, and wherein a first hose portion (10) of the end part extends from the basic body through substantially 180° of the circular curved part (9) and a second hose portion (11) extends through the remaining portion of the curved part.

## Patentansprüche

1. Angiographiekatheter mit einem steifen schlauchartigen Basiskörper (2) und einem weniger steifen schlauchartigen Endteil (6), das mit einem Ende des Basisteiles verbunden ist, wobei dieses Endteil eine gekrümmte Form aufweist,
dadurch gekennzeichnet, daß das Endteil aus mindestens zwei Schlauchabschnitten (10, 11) aus Material verschiedener Steifigkeiten zusammengesetzt ist, die miteinander in Längsrichtung verbunden sind.

2. Katheter nach Anspruch 1, bei dem die Steifigkeit des Materiales der Schlauchabschnitte (10, 11) in die Richtung von dem Basiskörper zu dem freien Ende hin abnimmt.

3. Katheter nach Anspruch 2 des "Schweineschwanz"-Types, bei dem die gekrümmte Form des Endteiles einen geraden Abschnitt, der mit dem Basisteil verbunden ist, und einen Kreisabschnitt, der im wesentlichen um mehr als 270 Grad gekrümmt ist und mit dem geraden Abschnitt verbunden ist, aufweist, und bei dem ein erster Schlauchabschnitt (10) des Endteiles sich von dem Basiskörper um im wesentlichen 180 Grad des gekrümmten Kreisteiles (9) erstreckt und ein zweiter Schlauchabschnitt (11) sich über den verbleibenden Abschnitt des gekrümmten Teiles erstreckt.

## Revendications

1. Cathéter angiographique comprenant un corps de base rigide (2) en forme de tuyau et une partie terminale (6) moins rigide, en forme de tuyau, raccordée à une extrémité de ce corps de base, dans lequel cette partie terminale a une forme recourbée, caractérisé en ce que ladite partie terminale est formée par l'assemblage d'au moins deux portions de tuyaux (10, 11) faites de matières possédant des rigidités différentes, et qui sont assemblées l'une à l'autre dans la direction longitudinale.

2. Cathéter selon la revendication 1, dans lequel la rigidité de la matière des portions de tuyaux (10, 11) décroît selon la direction du corps de base vers l'extrémité libre.

3. Cathéter selon la revendication 2, du type "queue de cochon", dans lequel la forme recourbée de la partie terminale comprend une portion droite qui est reliée au corps de base et une portion en arc de cercle recourbée sensiblement sur plus de 270°, reliée à la portion droite, et dans lequel une première portion de tuyau (10) de la partie terminale part du corps de base et s'étend sensiblement sur 180° de la partie recourbée en arc de cercle (9) et une deuxième portion de tuyau (11) s'étend sur la partie restante de ladite partie recourbée.
